# EUROPEAN PATENT APPLICATION

(11) **EP 3 103 865 A1**
(43) Date of publication of application: **14.12.2016**
(21) Application number: 15746514.7
(22) Date of filing: 15.01.2015
(51) Int. Cl.: C12N 5/071, A61L 27/60, A61F 2/10

(54) **METHOD FOR OBTAINING A SPRAY-ON CELLULAR COMPOUND OF HUMAN FIBROBLASTS AND KERATINOCYTES IN SOLUTION AND THE USE THEREOF AS A REGENERATIVE AGENT IN SKIN LESIONS**

(30) Priority: 04.02.2014 MX 2014001356
(71) Applicant: Alvaro Galue, Eduardo, Monterrey 64630 (MX)
(72) Inventor: Alvaro Galue, Eduardo, Monterrey 64630 (MX)
(74) Representative: ABG Patentes, S.L.
(86) International application number: PCT/MX2015/000001
(87) International publication number: WO 2015/119491

(57) **Abstract**

The present invention relates to a process for obtaining a compound of cellular spraying for fibroblasts and human keratinocytes and its use as regenerative agent of cutaneous lesions, mainly in diabetic ulcers, first, second, and third grade burns, and a substitute for the use of flaps techniques. The process for obtaining a compound of cellular spraying for fibroblasts and human keratinocytes includes the steps of separating the dermis and epidermis layers, incubation of the epidermis layer, disintegration of dermis layer and proliferation of fibroblasts, disintegration of the epidermis layer, proliferation of keratinocytes in suspension, preparation of the cross-linking reaction agent solution of the cellular spraying solution, and preparation of cellular spraying solution. The compound for cellular spraying of fibroblasts and human keratinocytes increases the application efficiency and ease of it, in order to be an economic alternative, fast, and easy to use.

## Description

### OBJECT OF THE INVENTION

The objective of the present invention is a process for obtaining a compound of cellular spraying for fibroblasts and human keratinocytes and its use as regenerative agent of cutaneous lesions, mainly in diabetic ulcers, first, second, and third grade burns, and a substitute for the use of flaps techniques. The compound for cellular spraying of fibroblasts and human keratinocytes increases the application efficiency and ease of it, in order to be an economic alternative, fast, and easy to use.

### BACKGROUND

Today the use of allogeneic grafts and xeno transplants limited the regeneration of the skin due to inflammatory processes that are caused by the immune rejection that involves the use of skin from donors and the use of fur from animals. This causes the low availability of such biological material for the treatment of skin wounds and there is the inconvenience of the susceptibility to infection. Currently there are patents of life human cell printing techniques called Bioprinting, where fibroblasts and keratinocytes are printed over the skin wounds. This technique is based on mechanically depositing by a robotic arm (with freedom to move in the 3 dimensional axes) autologous cells over the wound. Adding to this, it can be found in the market, dermal substitutes based on the in-vitro culture of human keratinocyte over arrays of skin de-cellularized of bovine origin. Use is only based on the increase in the time of the wound healing through the transplantation of this de-cellularized matrix of bovine origin with human keratinocytes previously planted on its surface.

However, besides using a product of animal origin (matrix de-cellularized) that is not 100% compatible with the human being, the use and transplant of this product is based only on increasing the use of the epithelization and wound regeneration and ignores the regeneration of the skin functionality to create again the pigmentation and the induction of hair. In addition to this, a severe trauma caused to this organ (skin) can cause a partial or incomplete regeneration and atrophy by the use of these meshes with keratinocytes and a total failure for the generation of color and hair. Another approach for the epithelization and regeneration of the wound that has been developed in the laboratory is based on the in-vitro culture of human keratinocyte on layers of fibroblasts from mouse embryos with a high degree of proliferation. This in-vitro system has disadvantages such as the low production of the extracellular matrix of the created bio-graft, a two-dimensional size very limited in thickness and support to cover the area damaged as a protective barrier to the wound, and above that, also ignores the functionality of skin regeneration during wound healing. (Pigmentation and induction of hair)

### DETAILED DESCRIPTION OF THE INVENTION

The present invention refers to a process for obtaining a compound of cellular spraying for fibroblasts and human keratinocytes, preferably fibroblasts and human autologous keratinocytes and/or allogeneic suspended in a solution and its use as
regenerative agent of cutaneous lesions, which comprises the following steps:

### (a) Separation of the dermis and epidermis.

Obtaining a skin sample of the patient skin under sterile conditions by means of a dermatome to obtain a skin sheet, preferably of 0.015 inch. After, wash the skin sample at least 3 times with a phosphates saline solution (PBS) 1X containing 10% v/v of an antibiotic/antimycotic solution. Then, wash at least 3 times with a PBS solution 1X containing 1% v/v of antibiotic/antimycotic. Preferably, the antibiotic/antimycotic solution is streptomycin/ penicillin. After the washing of the skin sample, incubating with a cell disintegration proteolytic enzyme, preferably dispase, at a concentration of 1.79 units/mg in conditions of temperature 4°C for at least 16 hours to obtain an epidermis layer and a dermis layer. After 16 hours, separate the two layers using sterile tweezers and place these layers in separated sterile containers.

### (b) Incubation epidermis layer to obtain a trypsinized epidermis.

Separating and washing the epidermis layer in a separate container at least 3 times with the PBS solution 1X and incubate the epidermis layer in a solution of 0.025% trypsin to a final volume of ethylene diamine tetraacetic acid (EDTA) for 20 minutes at a temperature of 37°C to obtain a trypsinized epidermis.

### (c) Disintegration of the dermis layer and proliferation of the fibroblasts

Place the dermis layer in a container and mechanically breaking down with a pulso cutting tool in sterile conditions to obtain pieces of dermis pieces with a maximum of 1 mm x 1 mm, and incubate for a period of at least 10 minutes, carefully adding 33.33% of the volume of the container of a cell culture medium for fibroblast to the walls of the container and avoid moving the dermis fragments and incubate at 37°C, 95% relative humidity, 5% CO₂ a 20% O₂ to obtain fibroblasts in a monolayer.

### (d) Breakdown of the layer of epidermis.

Neutralize the **trypsinized** epidermis with 50% of human keratinocytes culture medium containing 10% of fetal bovine serum, and vigorously shaking for at least 30 seconds to get a cellular solution. Then, filtering the cellular solution through a filter of nitrocellulose with a pore size of 40µm to obtain human keratinocytes in suspension in the filtered product.

### (e) Proliferation of the keratinocytes in suspension.

Plant the suspension of keratinocytes in the monolayer under aseptic conditions in a container with a cell culture medium and incubate at 37°C, 95% RH, 5% CO₂ and 20% O₂ for a period of 2 to 4 weeks under aseptic conditions, and change of cell culture medium every second or third day until reaching a 100% confluence of keratinocyte to obtain the most amount of keratinocytes in the monolayer.

### (f) Preparation of the solution of the cross-linking reaction agent of the cellular spraying solution

Generating in aseptic conditions 15ml of lung bovine thrombin at 27% v/v at a concentration of 11 U/ml in 0.9 sodium chloride 0.9% v/v, and charging 15ml of the cross-linking reaction agent in a 15cc syringe to obtain the cross-linking reaction agent of the cellular spraying solution.

### (g) Preparation of the cellular spraying solution

Centrifuging at 400g for 5 minutes a 50cc of autologous peripheral blood, preferably human platelets (coming from the patient sampling) or Cryo preserved platelet (blood bank) to obtain 15% v/v of acellular plasma serum and re-suspending with 10 x 10⁶ fibroblasts in monolayer and 10 x 10⁶ keratinocytes in the monolayer and charging 15ml of the cellular spraying solution in a 15cc syringe, and mixing the cellular spraying compound with the cellular spraying solution with the use of a fibrijet sprayer to obtain a cellular spraying compound of fibroblasts and human keratinocytes.

### EXAMPLE 1. PREFERABLY EMBODIMENT OF THE EMPLOYMENT OF THE CELLULAR SPAYING COMPOUND OF KERATINOCYTES AND HUMAN FIBROBLASTS ON SKIN LESIONS.

Connecting the 15cc syringe with the cellular spraying compound and the 15cc syringe, the cross-linking reaction agent of the spraying solution to the fibrijet sprayer. Connect the fibrijet sprayer to a compressed air regulator unit. Connect the compressed air regulator to a compressed air power unit to 25 PSI/1.75 bar. Place the fibrijet to a distance not less than 3 cm above the surface of the tissue.

Open the compressed air source and push the plunger of both syringes at the same time for the pulverization of the keratinocytes and human fibroblasts.

Alternatively, the cellular spraying compound of fibroblasts and human keratinocytes is prepared 15% v/v of a solution composed of 225 grams/ml of bovine blood Fibrinogen dissolved in a saline phosphate buffer at a concentration of 1x.

Alternatively, the cellular spraying fibroblasts human keratinocytes compound can be obtained using the product of Fibrinogen from human donor via the product called "Tissucol" of Baxter laboratories.

Alternatively, the cross-linking reaction agent of the spraying solution is obtained by loading a 15cc syringe with 1% v/v CaCl₂.

Alternatively, the cross-linking reaction agent of the spraying solution can be obtained using the human thrombin product from donor named "Tissucol" of Baxter laboratories.

### EXAMPLE 2. PREFERABLE MODE OF THE INVENTION

The cell autologous fibroblasts and human keratinocytes compound and/or the donor include a cellular spraying solution and a solution of the cross-linking reaction agent of the cellular spraying solution that the time of the pressure pulverization combined both molecules to encapsulate the living cells which will be deposited on the surface of the tissue and will form a film or gel filling with living cells in its interior to the generation of new extracellular matrix.

### EXAMPLE 3. MANIPULATION OF CELLULAR SPRAYING COMPOUND

In an operating room is performed the cellular spraying process by the simultaneous spraying of 2 solutions by a medical device (fibrijet-micromedics gas applicator) to generate a gelling film and/or filling onto the wound or skin lesion as re-epithelization treatment and empowering of the regeneration of the skin through encapsulation of living cells on the gelling film.

### EXAMPLE 4. PREFERENTIAL USE OF THE INVENTION.

In an operating room, the cellular spraying process is performed that includes 2 solutions sprayed at the same time in a medical device (fibrijet-micromedics gas applicator) to generate a gelling film and/or filling onto the wound or skin lesion with living cells encapsulated as re-epithelization treatment.

The medical device (fibrijet-micromedics gas applicator) contained two 5cc syringes with the two reacting solutions: cellular spraying solution (Fibrinogen, fibroblasts, and keratinocytes) and the cross-linking reaction agent solution (thrombin) of the cellular spraying solution.

Spraying will be done directly on the cutaneous wound at least 3 cm away through medical device (fibrijet-micromedics gas applicator) connected to a compressed air supply. The pressure required to exercise the spraying will be 25 PSI 1.75 bar of compressed air that pushed the two syringes and sprayed their contents with fine droplets on the wound getting a gel-forming film of fibrin containing fibroblasts and keratinocytes that will promote the production of a new extracellular matrix on the wound. To obtain a coarse droplets spray process, the spraying will be of 15 PSI / 1.0 bar of compressed air. Therefore, this process will produce a re-epithelization of the wound in a short time, a reorganization of the tissue, and an early regeneration of the lesion avoiding exposure to infections.

The cellular spraying compound of fibroblasts and human keratinocytes that is in contact with the skin lesion, provide with the passage of time a re-epithelization, and a faster regeneration because of the addition of living fibroblasts embedded within this network of fibrin (human Fibrinogen, human acellular plasma, bovine Fibrinogen) stimulating the new generation of extracellular matrix and collagen fibers.

Having described enough my invention, consider it as a novelty and therefore claim as my exclusive property, contained in the following claims.

## Claims

1. A process for obtaining a compound of cellular spraying for fibroblasts and keratinocytes comprising the steps of:
a) separation of a dermis layer and an epidermis layer;
b) incubating of the epidermis layer to obtain a trypsinized epidermis;
c) disintegrating the dermis layer and proliferating fibroblasts;
d) disaggregating the epidermis layer;
e) proliferating keratinocytes in suspension;
f) preparing a cross-linking reaction agent solution of the cellular spraying solution; and
g) preparing of cellular spraying solution.

2. The process for obtaining a compound of cellular spraying for fibroblasts and keratinocytes according to claim 1, wherein the step a) a sample of skin is obtained from a patient under sterile conditions by using a dermatome to obtain a skin sheet; washing the skin sheet at least 3 times with a saline solution of phosphates (PBS) 1X containing 10% v/v of antibiotic/antimiotic; washing at least 3 times with a PBS 1X solution containing 1% v/v of antibiotic/antimiotic; incubating with a cellular disintegration proteolytic enzyme at a concentration of 1.79 units/mg at a temperature of 4°C for at least 16 hours to get the epidermis layer and the dermis layer; after 16 hours, separating the two layers using sterile tweezers and placing the layers in separate and sterile containers.

3. The process for obtaining a compound of cellular spraying for fibroblasts and keratinocytes according to claim 1, wherein skin sheet has a size of 0.015 inch.

4. The process for obtaining a compound of cellular spraying for fibroblasts and keratinocytes according to claim 1, wherein the antibiotic/antimiotic is penicillin/streptomycin.

5. The process for obtaining a compound of cellular spraying for fibroblasts and keratinocytes according to claim 1, wherein the enzyme is dispase.

6. The process for obtaining a compound of cellular spraying for fibroblasts and keratinocytes according to claim 1, wherein the step b) includes separating and washing the epidermis layer in the separate container at least 3 times with the PBS solution 1X and then incubating the epidermis layer in a 0.025% trypsin solution in a final volume of ethylene diamine tetraacetic acid (EDTA) for 20 minutes at a temperature of 37°C to obtain a trypsinized epidermis.

7. The process for obtaining a compound of cellular spraying for fibroblasts and keratinocytes according to claim 1, wherein the step c) includes placing the dermis layer in the container and mechanically disintegrating with a constant pulse tool in sterile conditions to obtain dermis pieces with a maximum size of 1 mm x 1 mm, and then incubating for a period of at least 10 minutes, and adding to walls of the container 33.33% of the volume of the container of a cell culture medium for fibroblast and preventing moving the dermis pieces, then incubating at 37°C, 95% RH, and 5% CO₂ and 20% O₂ to obtain a monolayer of fibroblasts.

8. The process for obtaining a compound of cellular spraying for fibroblasts and keratinocytes according to claim 1, wherein the step d) includes neutralizing the trypsinized epidermis with 50% of human keratinocytes culture medium containing 10% fetal bovine serum, and stirring vigorously for at least 30 seconds to produce a cell solution, then filtering the cell solution through a filter of nitrocellulose with a pore size of 40µm to obtain human keratinocytes in suspension in the filtered product.

9. The process for obtaining a compound of cellular spraying for fibroblasts and keratinocytes according to claim 1, wherein the step e) including planting the keratinocytes monolayer under aseptic conditions in a container with a cell culture medium and incubating at 37°C, 95% relative humidity, 5% CO2 and 20% O₂ for a period of 2 to 4 weeks, and changing the cell culture medium every second or third day to reach a 100% of confluence of keratinocyte to obtain a large amount of keratinocytes in the monolayer.

10. The process for obtaining a compound of cellular spraying for fibroblasts and keratinocytes according to claim 1, wherein the step f) includes generating in aseptic conditions 15ml of 27% v/v lung bovine thrombin at a concentration of 11 U/ml in 0.9 % v/v of sodium chloride and charging a syringe with 15ml of the cross-linking reaction agent to obtain the cross-linking reaction agent of the cellular spraying solution.

11. The process for obtaining a compound of cellular spraying for fibroblasts and keratinocytes according to claim 1, wherein the step g) includes spinning at 400g for 5 minutes a 50cc of autologous peripheral blood to obtain 15% v/v of acellular plasma serum and then re-suspending with 10 x 10⁶ fibroblasts in monolayer and 10 x 10⁶ keratinocytes in monolayer, and loading 15ml of the cellular spraying solution in a 15cc syringe of 15cc, and then mixing the cellular spraying compound with the cellular spraying solution in a fibrijet sprayer to obtain the compound of cellular spraying for fibroblasts and keratinocytes.

12. The process for obtaining a compound of cellular spraying for fibroblasts and keratinocytes according to claim 11, wherein the autologous peripheral blood is human platelets or preserved cryo platelet.

13. The process for obtaining a compound of cellular spraying for fibroblasts and keratinocytes according to claims from 1 to 12 as regenerative agent for skin lesions.
